(19)

Europäisches Patentamt
European Patent Office
Office européen des brevets

(11) **EP 3 929 929 A1**

(12) **EUROPEAN PATENT APPLICATION**

(43) Date of publication:
**29.12.2021 Bulletin 2021/52**

(51) Int Cl.:
***G16H 10/40*** (2018.01)  ***G16H 10/60*** (2018.01)

(21) Application number: **20182720.1**

(22) Date of filing: **26.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Medicus AI GmbH**
**1060 Wien (AT)**

(72) Inventors:
• **NEHME, Nadine**
  **1060 Wien (AT)**

• **AL HAKIM, Baher**
  **1060 Wien (AT)**
• **KAWAS, Mouhamad**
  **1060 Wien (AT)**
• **ALKHATIB, Bassel**
  **1060 Wien (AT)**

(74) Representative: **Stellbrink & Partner**
**Patentanwälte mbB**
**Widenmayerstrasse 10**
**80538 München (DE)**

(54) **SYSTEM AND METHOD FOR SAMPLE ANALYSIS**

(57)    Disclosed is a system, comprising a modification node and at least two analysis nodes. The system is configured for receiving analysis instruction data. The analysis nodes are configured for generating analysis results for a plurality of samples. The modification node is configured for generating modified analysis result data corresponding to the analysis results, and for sending portions of the modified analysis result data to recipient nodes. Further disclosed is a corresponding method together with a corresponding computer program product.

Fig. 1

EP 3 929 929 A1

**Description**

**Field**

The present invention relates to the field of sample analysis of biological and/or medical samples. The present invention further relates to interoperability of different systems for sample analysis.

Currently, medical laboratories operate with proprietary or standardized instructions for analyses to be performed with medical samples. For example, the LOINC standard allows for a standardized exchange of data relating to analyses or measurements relating to medical samples. However, there is a manifold of other standardized or proprietary standards. Also, different calibrations and analysis procedures can lead to different results of analyses of a same sample or samples comprising same properties concerning the analysis in question.

Also, for further processing by a health care provider, analysis results are often compared to ranges relating to a "standard population" or assigned to groups of samples, such as "high", "medium" and "low" groups.

For a recipient of an analysis result, it is often unclear to which standard population the groups or the comparison result relates. Often, the comparison is performed by a data-processing system within a laboratory performing the analysis. It can for example be a standard setting of an analysis machine.

The above-discussed laboratory-specific data can often not be modified without technical efforts, for example because they are hardcoded in a machine, or because they are pre-configured and other values are not available, for example due to privacy concerns. Also, different recipients of analysis results typically work with comparison results made under one set of settings.

Hence, an analysis result generated for one recipient, for example one health care provider, may not be useful or at least not ergonomic for use by another health care provider. Also, systems of the health care providers can be specific to sets of settings, for example clinical decision support systems, or patient record systems. Also, analysis data in records may be useless if the above-discussed settings were different for the analysis of different samples.

Furthermore, it may not always be known which health care providers will need the analysis results relating to a sample, since a patient may be treated by several health care providers for a same condition, e.g. first by a general practitioner and then by a specialist. This can, inter alia due to the potential above-mentioned incompatibilities, lead to further tests of samples from a same patient without a clinical necessity or to a further, substantially identical analysis of a sample taken from the patient.

**Summary**

It is therefore an object of the invention to overcome or at least alleviate the shortcomings and disadvantages of the prior art. More particularly, it is an object of the present invention to provide an improved system and method for sample analysis.

It is an optional object of the present invention to provide a system and method for providing ergonomically improved results of sample analyses.

It is another optional object of the present invention to provide a system and method for an improved interoperability of laboratory systems for medical laboratories and systems of recipients of analysis results from sample analyses of medical laboratories.

In a first embodiment, a system is disclosed.

The system comprises a modification node and at least two analysis nodes.

The modification node may comprise a data-processing system. The modification node and/or the data-processing system may comprise at least one or a plurality of components. At least some of the components may be software components, such as parts of a software or different programs interacting.

The system may be configured for receiving analysis instruction data.

The analysis nodes may be configured for receiving at least a part of the analysis instruction data. In other words, each of the analysis nodes may be configured for receiving a part of the analysis instruction data. The parts may be same parts. The parts may also overlap. The parts may also be completely different.

The analysis nodes may be configured for generating analysis results for a plurality of samples.

It is to be noted that the analysis results intended to refer to results of measuring in the classical sense, but for example also to chemical, bio-chemical or biologic analysis of an object, which generates information about at least one physical, chemical, biological or bio-medical feature of a sample. The analysis may be a direct measurement direct, such as measuring a temperature by means of a sensing unit configured for sensing the temperature. The analysis may also refer to indirectly determining data based on sensed data, for example, an acceleration may be indirectly measured by measuring a corresponding velocity and generating a derivate with respect to the time, or by an adapted sensing unit that senses a force of an object with known inertia against the accelerated object. The analysis may also relate to determining a concentration of a substance A in a mixture B. This may for example be the case when measuring a concentration of cholesterol in blood of a user.

Each of the analysis nodes may comprise a set of analysis equipment. The set of analysis equipment can comprise devices, systems and/or apparatuses configured for performing at least one or a plurality of analyses.

The modification node may be configured for generating modified analysis result data corresponding to the analysis results. In other words, the modification node may be configured for modifying the analysis results and thus generating the modified analysis result data. The modification node may also be configured for generating analysis result data that are a modified version of the analysis results.

The system may be configured for sending portions of the modified analysis result data to recipient nodes.

The modification node may be configured for sending portions of the modified analysis result data to the recipient nodes.

The analysis instruction data may comprise at least one of analysis instructions and recipient node data.

The analysis instructions may be instructions to perform at least one or a plurality of analyses. The analysis instructions may for example relate to an analysis to be performed, a type of a sample to be analyzed, a preparation of the sample and/or a variable to be determined, such as a concentration. The analysis instructions may be specific to one or more analysis nodes.

The analysis instructions may be human-intelligible. They may also be machine-interpretable.

The recipient node data may refer to a corresponding recipient node. For example, the recipient node data may refer to a recipient node that send a portion of the analysis instruction data. The recipient node data may also refer to a recipient node which took a certain sample or a plurality of samples from at least one or a plurality of users. The recipient node data may also refer to a recipient node to which an analysis result or a portion of the modified analysis result data is to be sent.

The analysis instruction data may comprise sample data. The sample data may comprise portions that each relate to single samples, for example to an identification of a sample, such as an identification element of a packaging of the sample. The sample data or the portions thereof may for example also relate to a type of samples.

The analysis nodes may be configured for generating the analysis results based on at least a part of the analysis instruction data. For example, the analysis nodes may be configured for automatically generate the analysis results. The analysis nodes may be configured for generating the analysis results based on input data or transmitted data, which may be generated by the respective set of analysis equipment.

The analysis results generated by the analysis nodes may each be initially according to at least one respective initial analysis reference. In other words, each analysis result may correspond to at least one initial analysis reference. However, a plurality of analysis results can correspond to a same initial reference.

When reference is made to "each analysis result", the person skilled in the art will easily understand that this refers to the analysis results that the system is configured to apply an inventive process. However, the system may be configured for processing or generating other analysis results, for example in ways known in the art, or without application of the inventive process or without at least one part of the inventive process.

At least two analysis results may be according to different initial analysis references. In other words, the analysis nodes may be configured to for generating analysis results according to different initial analysis references. For example, two analysis nodes can be configured for using different initial analysis references, so that the analysis results that they are configured to generate are according to different initial analysis references.

Each analysis node may be configured for using at least one initial analysis reference. At least two analysis nodes may be configured for generating analysis results according to different initial analysis references.

The initial analysis references may be entirely different. For example, an analysis node A may be configured for using an initial analysis reference $\alpha$ and for generating analysis results according to the initial reference $\alpha$. An analysis node B may be configured for using an initial analysis reference $\beta$ and for generating analysis results according to the initial reference $\beta$.

However, the initial analysis references may also only be partially different, for example in a case where the analysis node A may be configured for using the initial analysis reference $\alpha$ and initial analysis references $\gamma$ and $\delta$, and wherein the analysis node B may be configured for using the initial analysis references $\beta$, $\gamma$ and $\delta$. In such a case, the analysis node A is still configured for generating analysis results according to the initial analysis reference $\alpha$, which is different from the initial analysis references to which analysis results of the analysis node B may be according.

The modification node may be configured for generating the modified analysis results based on at least one of the recipient node data and the sample data.

For example, the recipient node data may comprise an indication of at least one analysis reference corresponding to a recipient node. In such a case, the modification node may be configured for generating the modified analysis results according to said analysis reference. In another example, the sample data may comprise an indication of at least one analysis reference, and the modification node may be configured for generating the modified analysis results according to said analysis reference.

The system, preferably the modification node, may be configured for sending the portions of the modified analysis result data to the recipient nodes based on the recipient node data. For example, the recipient node data may indicate a type of transmission or data that the recipient node can receive and/or process.

The system and/or the modification node may be configured for generating modified analysis results at least for each analysis result for which an output analysis reference and the initial analysis reference are different.

The respective output analysis reference may be an analysis reference to which the analysis result has to be according for further processing, for example for processing by the recipient node.

The system may be configured for selecting the output analysis reference for each analysis result based on at least one of the analysis instruction data and the recipient node data. The modification node may be configured for selecting the output analysis reference for each analysis result based on at least one of the analysis instruction data and the recipient node data.

As discussed above, the recipient node data may comprise an indication of at least one analysis reference corresponding to a recipient node. Said at least one analysis reference may for example be at least one analysis reference that the recipient node may be configured to process.

The analysis instruction data may for example comprise an indication of an output analysis reference.

These options may be optionally advantageous, as they may allow generate the analysis results by analysis nodes using different analysis references as recipient nodes. Thus, an overall system can for example assign analyses of samples in a more flexible way to analysis nodes. Also, analysis results from different analysis nodes may thus be usable for more recipient nodes. This may for example be relevant when an analyses for a first sample that was initially generated for a first recipient node becomes relevant for a second recipient node. A practical example may be a transfer of a patient from one hospital to another, or a referral from a general practitioner to a specialist.

The system and/or the modification node may further be configured for selecting the output analysis reference for each analysis result based on the sample data.

For example, the sample data may indicate an output analysis reference. The sample data may also indicate a recipient node and the at least one analysis reference that the recipient node is configured to process.

The modification node can comprise an output reference selector component. The output reference selector component can be configured for selecting the output analysis reference.

The output reference selector component may be implemented in software. Thus, the output reference selector component may be a software component, or at least a portion of one or more software components. The modification node and/or the data-processing system of the modification node may be configured for running said software component, and/or for running a software comprising this software component.

The output reference selector component may be stored on one or more different storage devices. For example, the output reference selector component may be stored on a plurality of storage components comprising persistent memory, for example a plurality of storage devices in a RAID-system, or different types of memory, such as persistent memory (e.g. HDD, SDD, flash memory) and main memory (e.g. RAM).

The output reference selector component may also be implemented at least partially in hardware. For example, the output reference selector component can be a processing unit or a system-on-chip that can be interfaced with the modification node, the data-processing system, a personal computer, a laptop, a pocket computer, a smartphone, a tablet computer and/or a server.

The output reference selector component may also comprise elements implemented in hardware and elements implemented in software. An example may be a use of a hardware-implemented encryption/decryption unit and a software implemented processing of the decrypted data.

The output reference selector component may comprise elements specific to the modification node and/or the data-processing system, for example relating to an operating system, other components of the modification node, or recipient nodes to which the modification node can be connected.

Each analysis result value may comprise a numeric result value. That is, each analysis result value may comprise a value that may be stored as numeric value. For example, the numeric value can be a quantitative indication. The analysis result value may comprise further values, or example a qualitative indication, a unit, a name or indication of the corresponding analysis, or the like. The analysis result value may however also consist in the numeric result value.

However, the person skilled in the art will easily understand that there can still be other result values that are processed, but that there can be a group of analysis result values in which each analysis result value comprises a numeric result value.

Each analysis result may be initially associated with a segment of a respective initial result range. That is, each analysis result may initially relate to a segment of such an initial result range. For example, the segment may comprise the numeric result value of the analysis result value.

Each initial result range may be corresponding to an initial analysis reference. For example, an analysis reference, which can be the initial analysis reference, may comprise at least one result range. The result range may for example comprise a maximum and a minimum value that an analysis can yield, such as 0% up to 100%. The result range may also be a scale of result values, such as a scale for a length, e.g. in mm, a temperature, e.g. in °C or a concentration, e.g. in mg/ml, mg/g, mol/ml or another indication of a concentration. In case that the exemplary analysis reference is the initial analysis reference, the initial analysis reference may comprise the initial result range.

Generating the modified analysis results may comprise for at least some of the analysis results, associating the analysis results to corresponding segments of an output result range. In other words, generating the modified analysis results may comprise associating the analysis results to one or more segments of the output result range, so that the analysis results are associated to segments of the output result range to which they correspond.

The modification node may comprise a range association component. The range association component may be configured for associating at least some of the analysis results to the corresponding segments of the output result range.

The modification node may comprise a range association component. The range association component may be configured for selecting the output analysis reference.

The range association component may be implemented in software. Thus, the range association component may be a software component, or at least a portion of one or more software components. The modification node may be configured for running said software component, and/or for running a software comprising this software component.

The range association component may be stored on one or more different storage devices. For example, the range association component may be stored on a plurality of storage components comprising persistent memory, for example a plurality of storage devices in a RAID-system, or different types of memory, such as persistent memory (e.g. HDD, SDD, flash memory) and main memory (e.g. RAM).

The range association component may also be implemented at least partially in hardware. For example, the range association component can be a processing unit or a system-on-chip that can be interfaced with the modification node, a personal computer, a laptop, a pocket computer, a smartphone, a tablet computer and/or a server.

The range association component may also comprise elements implemented in hardware and elements implemented in software. An example may be a use of a hardware-implemented encryption/decryption unit and a software implemented processing of the decrypted data.

The range association component may comprise elements specific to the modification node, for example relating to an operating system, other components of the modification node, or recipient nodes to which the modification node can be connected.

Each output result range may correspond to an output analysis reference.

The output result range may comprise a same or a different dimension as the input result range. For example, depending on an analysis method, different (numeric) values for a same sample in a same unit may be generated due to the different methods.

Generating the modified analysis results may further comprise for the at least some of the analysis results modifying the numeric result value so as to maintain a same relative position in the segment of the output result range as within the segment of the initial result range.

For example, when a segment A of the input result range comprises an interval [a, b], and a segment B of the output result range comprises an interval [c, b]. Then, a result comprising the numeric result value X in the segment A may be modified so as to comprise a numeric value Y in the segment B, wherein $\frac{X-a}{b-a} = \frac{Y-c}{d-c}$. Thus, the relative position of the result within the result range can be maintained.

An optional advantage can be that for this modification, it may be not necessary to have further empiric information relating to the respective analysis or the respective analysis results. Furthermore, the modification may optionally advantageously require only few computational resources.

However, the modification may yield data according to the output result range. This may be optionally advantageous, as it may render data usable, particularly when data that initially comprised different analysis references are to be processed, or when the data are to be processed by different systems using different analysis references, as discussed above.

The same relative position may be a position in a same quantile of a distribution of analysis results in the segments of the output result range and the input result range. In other words, modifying the analysis results may comprise associating a result to a quantile of a distribution in the output result range, to which quantile it corresponded in the input result range. The quantile may be a quantile of a distribution of result values. For example, if a result corresponds to a quantile comprising lowest 5% to lowest 10% of result values in the input result range, then the result an correspond to a same quantile in the output result range.

An optional advantage can be that the modified result data may not distort a distribution of data associated with the output result range. This may particularly be the case when a "true" correspondence of analysis results according to the initial analysis reference and the output reference or the initial result range and the output result range are not known or cannot be estimated efficiently. This can be particularly the case with a plurality of variables, which may require multivariate optimisation. The latter may require considerable amounts of computational resources.

Generating the modified analysis results may comprise for the at least some of the analysis results, transforming the numeric result value from the segment of the initial result range to the segment of the output result range by means of an affine transformation. An optional advantage may be that an affine transformation may be computationally less demanding and does not require an availability of empirical data, e.g. regarding an exact or estimated correspondence of analysis results in different result ranges or a distribution of the analysis results.

The modification node and/or the data-processing system may be configured associating the analysis results to the segment of the output result range. The range association component may be configured for associating the analysis results to the segment of the output result range.

The modification node may be configured for associating results from a same segment of the initial result range to a same segment of the output result range. The range association component may be configured for associating results from a same segment of the initial result range to a same segment of the output result range.

The system and/or the modification node may be configured for associating results from a same segment of the initial result range to a same segment of the output result range if a number of segments of the output result range equals a number of segments of the initial result range. In other words, in case of a same number of segments in the output result range and in the initial result range, the system and/or the modification node may be configured to associate analysis results from same segments of the initial result range to same segments of the output result range, for example from a segment A of the input result range to a segment $\alpha$ of the output result range, from a segment B of the input result range to a segment $\beta$ of the output result range etc.

Thus, optionally advantageously, information associated with the segments may be conserved and available also in the modified analysis results.

Associating the analysis results to the segments of the output result range may comprise associating results from a plurality of segments of the initial result range to one segment of the output result range. This may be the case if the initial result range comprises more segments than the output result range.

In other words, in a case where the input result range comprises more segments than the output result range, analysis results from more than one segment of the input result range may be assigned to at least one or one segment of the output result range.

Associating the analysis results to the segment of the output result range, respectively, may comprise associating results from one or at least one segment of the initial result range to a plurality of segments of the output result range, preferably if the initial result range comprises less segments than the output result range.

In other words, in a case where the input result range comprise less segments than the output result range, analysis results from one or at least one segment of the input result range may be assigned to more than one segment of the output result range.

These two options may be optionally advantageous to conserve information associated with the segments of the result ranges.

Associating the analysis results to the segment of the output result range, respectively, may comprise associating results from same continuous intervals of the one or at least one segment of the initial result range to same segments of the output result range. For example, analysis results in an interval [1.5, 2.5] of the input result range may be associated to a segment A of the output result range, and analysis results in an interval [2.5, 3.5] of the input result range may be associated to a segment B of the output result range.

The system may be configured for determining the segment of the output result range based on a pair of the initial analysis reference and the output analysis reference and by processing model data. The modification node may be configured for determining the segment of the output result range based on a pair of the initial analysis reference and the output analysis reference and by processing model data.

In other words, the model data may be configured to yield for input comprising an analysis result, the initial analysis reference and the output analysis reference a segment in of the output analysis reference.

The model data may comprise a rule set. The mode data may comprise model data of a machine learning model or at least one neural network, to that the model data can be used as estimator.

Generating the modified analysis result data may comprise comparing each of the analysis results to a comparison sample group. The comparison sample group may comprise samples for comparison.

The modification node may comprise a sample comparison component. The sample comparison component may be configured for comparing at least some or each of the analysis results to the comparison sample group.

The modification node can comprise an sample comparison component. More particularly, the modification node can comprise at least one storage device wherein the sample comparison component may be stored.

The sample comparison component may be implemented in software. Thus, the sample comparison component may be a software component, or at least a portion of one or more software components. The modification node may be configured for running said software component, and/or for running a software comprising this software component. In other words, the sample comparison component may comprise one or more computer instructions (i.e. machine-readable instructions) which can be executed by a computer (e.g. the modification node).

The sample comparison component may be stored on one or more different storage devices. For example, the sample comparison component may be stored on a plurality of storage components comprising persistent memory, for example a plurality of storage devices in a RAID-system, or different types of memory, such as persistent memory (e.g. HDD, SDD, flash memory) and main memory (e.g. RAM).

The sample comparison component may also be implemented at least partially in hardware. For example, the sample comparison component or at least a portion of the sample comparison component can be implemented as a programmed and/or customized processing unit, hardware accelerator, or a system-on-chip that can be interfaced with the modification node, a personal computer, a laptop, a pocket computer, a smartphone, a tablet computer and/or a server.

The sample comparison component may also comprise elements implemented in hardware and elements implemented in software. An example may be a use of a hardware-implemented encryption/decryption unit and a software implemented processing of the decrypted data.

The sample comparison component may comprise elements specific to the modification node, for example relating to an operating system, other components of the modification node, or recipient nodes to which the modification node can be connected.

Comparing the analysis results to the comparison sample group may comprise estimating a relative position of the respective analysis result in the corresponding comparison sample group.

The sample comparison component may be configured for storing data configured for estimating the relative position of an analysis result in the comparison sample group, for example a hash table associating analysis results and relative positions, or a sorted list of at least a portion of the analysis results, e.g. their numeric value.

The relative position in the comparison group may be a position in a quantile of the comparison sample group. In other words, the relative position may be a quantile in the sample comparison group within which an analysis result falls. The system, the modification node and/or the sample comparison component may be configured determining said quantile.

The relative position of an analysis result in the comparison group may be a segment of results in the comparison sample group in which the respective analysis result falls.

The system and/or the modification node may further configured for selecting the comparison sample group for each analysis result or for each of at least some of the analysis results. In other words, at least one of the system and the modification node may be configured for selecting comparison sample groups for the analysis results.

The modification node may further comprise a selector component. The selector component may be configured for selecting the comparison sample group for each analysis result.

The modification node can comprise an selector component. More particularly, the modification node can comprise at least one storage device wherein the selector component may be stored.

The selector component may be implemented in software. Thus, the selector component may be a software component, or at least a portion of one or more software components. The modification node may be configured for running said software component, and/or for running a software comprising this software component. In other words, the selector component may comprise one or more computer instructions (i.e. machine-readable instructions) which can be executed by a computer (e.g. the modification node).

The selector component may be stored on one or more different storage devices. For example, the selector component may be stored on a plurality of storage components comprising persistent memory, for example a plurality of storage devices in a RAID-system, or different types of memory, such as persistent memory (e.g. HDD, SDD, flash memory) and main memory (e.g. RAM).

The selector component may also be implemented at least partially in hardware. For example, the selector component or at least a portion of the selector component can be implemented as a programmed and/or customized processing unit, hardware accelerator, or a system-on-chip that can be interfaced with the modification node, a personal computer, a laptop, a pocket computer, a smartphone, a tablet computer and/or a server.

The selector component may also comprise elements implemented in hardware and elements implemented in software. An example may be a use of a hardware-implemented encryption/decryption unit and a software implemented processing of the decrypted data.

The selector component may comprise elements specific to the modification node, for example relating to an operating system, other components of the modification node, or recipient nodes to which the modification node can be connected.

Selecting the comparison sample group for each analysis result may be based at least on the sample data. For example, selecting the comparison sample group for each analysis result may be based on a type the samples.

The sample data may comprise data relating to each of the plurality of samples.

The samples may be samples originating from the human body.

For example, the samples may comprise samples of a bodily fluid, such as blood, urine or saliva. The samples may for example also comprise samples of at least one tissue from the human body, such as from a biopsy.

The analysis results may relate to bio-medical properties of the samples.

The analysis results relate to at least one or more quantitative analyses. A quantitative analysis may be configured for generating at least one quantitative result.

The sample data may comprise sample identification data of the samples. The sample identification data may be configured for identifying the samples.

The sample identification data may for example comprise an identification number, an index, or an identifier comprising an indication of a user from whom the sample originates. The sample identification data may for example also comprise an identification of a health care provider, an entity or a practitioner who took the sample.

For example, the sample identification data may relate to an index on a container or packaging of the sample, as discussed e.g. in WO98/05427 or EP 1 803 11.

The sample data may comprise user data relating to the users from whom the samples originate.

The user data may be pseudonymized, i.e. the user data may comprise a pseudonym of the patient. The user data may comprise an identification number that a third party such as a health care provider can match to the patient.

The user data comprise demographic data of the users, such as an age of the users, a sex of the users, a place of residence and/or an occupation of the users.

The user data comprise data relating to an age of the users.

The user data comprise data relating to a sex of the users.

The user data comprise genomic data of the users. That is, the user data may comprise data relating to a genome of the users, or to a part thereof.

The user data may comprise data relating to an ethnicity of the users.

The user data may comprise data relating to an environment of the users. The environment may be an environment in which the users live, or an environment in which the users work.

The user data may comprise data relating to a geographic environment in which the users live.

The user data may comprise data relating to an occupation of the users.

The user data may comprise portions relating to existing medical conditions of the respective users.

The user data may comprise portions relating to medications of the users. The portions of the user data relating to the medications of the users may for example relate to at least one or a plurality of

- pharmacologically active substances that are consumed,
- a dose or schedule of the medication,
- an administration of the substances, and
- a compliance of the users to the respective medication.

The user data may comprise portions relating to a drug consumption of the respective users. The term "drug consumption" is intended to refer to a consumption of drugs like alcohol, cigarettes, THC-containing products, or other drugs.

The portions relating to the drug consumption may for example relate to a consumed substance, an amount and/or a regularity of consumption.

Selecting the comparison sample group for each analysis result may be based at least on the user data relating to the users from whom the samples originate.

In other words, the comparison sample groups to which the samples may be compared may be selected based on the user data relating to the respective samples. For example, for a sample originating from a female user, a comparison sample group relating to analysis results from samples from other female users may be selected.

The above features to which at least a portion of the user data may relate may impact values that are determined by bio-medical analyses. Thus, comparing an analysis result of a user to analysis results of users sharing one of these features with the user may optionally advantageously allow to compensate for an influence of said feature. Thus, selecting the comparison sample group based at least one the user data relating to the users from whom the samples originate can be optionally advantageous for the same reason.

The sample comparison groups may comprise analysis results of samples corresponding to similar user data. The sample comparison groups may comprise portions of these analysis results, for examples the numeric result values of the analysis results.

The similar user data may be similar to each other according to a similarity measure.

The selector component may be configured for applying the similarity measure.

At least two of the sample comparison groups may each comprise analysis results corresponding to samples from to users of a same sex. In other words, there may be at least a sample comparison group A, comprising analysis results or portions thereof corresponding to samples of users of a sex $\alpha$, such as female, and a sample comparison group B, comprising analysis results or portions thereof corresponding to samples of a sex $\beta$, such as male. In some embodiments, the sample comparison groups may not comprise analysis results relating to samples from a user of the respectively opposite sex.

At least two of the sample comparison groups may each comprise analysis results corresponding to samples from users of a similar ethnicity. In other words, there may be at least a sample comparison group for samples originating from users of first ethnicity, and at least another sample comparison group for samples originating from users of a second ethnicity.

At least two of the sample comparison groups may each comprise analysis results corresponding to samples from users whose user data comprise similar portions similar medical conditions. Similar medical conditions may for example be same medical conditions. For example, there may be a comparison sample group for users having a first medical condition, such as diabetes type I, and a second comparison group for users having a second medical condition, such as high blood pressure. More than one sample comparison group may comprise at least a portion of a same analysis result relating to a same sample, e.g. if a user had more than one medical condition.

At least two of the sample comparison groups may each comprise analysis results corresponding to samples from users whose user data comprise similar portions relating to the medication. Similar portions relating to the medication may for example indicate a same medication, a medication with at least a same substance, or a medication with at least a substance from a same pharmacological family.

At least two of the sample comparison groups may each comprise analysis results corresponding to samples from users whose user data comprise similar portions relating to the drug consumption. Similar portions relating to the drug consumption may for example indicate a same drug consumption, a consumption of at least a same drug, or the like.

The person skilled in the art will easily understand that analysis results or portions thereof, which the sample comparison groups comprise, can be associated with a plurality of sample comparison groups. For example, an analysis result can be associated with a first sample comparison group relating to a sex of the respective user, and to another sample comparison relating to an ethnicity of the respective user.

The system and/or the modification node may be configured for generating a similarity by means of the similarity measure at least based on at least one of the demographic data of the users, the data relating to the ethnicity of the users, the data relating to the environment of the users and the data relating existing medical conditions of the users.

The system and/or the modification node may be configured for generating the similarity by means of the similarity measure at least based on the data relating to the medications of the users.

The system and/or the modification node may be configured for generating the similarity by means of the similarity measure at least based on the data relating to the drug consumption of the users.

The recipient node data may comprise for each sample an indication of a recipient node.

The indications of the recipient node may comprise an indication of an address associated with a corresponding recipient node data-processing system, such as an IP-address, an e-mail address, an ID, an authentication token, a hash of any of these, or the like.

Each of the recipient nodes may comprise the corresponding recipient node data-processing system.

The system may comprise the recipient nodes.

The analysis instructions may comprise instructions for analysing each of the samples.

The analysis instructions may comprise instructions for performing at least one bio-medical analysis for each sample.

The analysis instructions may comprise at least a machine interpretable portion.

The analysis results may be bio-medical analysis results.

The sets of analysis equipment may be configured for generating analysis data. The sets of analysis equipment may comprise devices configured for performing at least one bio-medical analysis, such as a centrifuge or a thermal cycler. The at least one bio-medical analysis is to be understood as at least one type of bio-medical analysis.

Each analysis node may comprise an analysis node data processing system. The analysis nodes may be configured for transmitting the analysis data to corresponding analysis node data processing systems.

The analysis data may be machine-interpretable or machine-readable. However, there can also be an agent instructed to enter the analysis data into a data-processing system of the corresponding analysis node, such as a laboratory information system. For example, the analysis node data processing systems may comprise interfaces enabling input of the analysis data.

The analysis nodes may be configured for processing the analysis data, for example so as to generate the analysis results.

The analysis node data processing systems may be configured for processing the analysis data.

The analysis nodes may be configured for generating the analysis results based on the analysis data.

For example, analysis data can be matched to corresponding sample data. The analysis data can also be combined with other sample data. The analysis data can be further processed, such as converted to another unit or compared to other data, such as prior analysis results. In another example, a format of the analysis data may be changed, e.g. by changing a header of a file comprising the analysis data, by changing a notation, or the like.

In a second embodiment, a method is disclosed. Above explanations, details, definitions and advantages can respectively apply.

The method comprises receiving the analysis instruction data. The method further comprises generating the analysis results for a plurality of samples by the at least two analysis nodes. Each of the analysis nodes comprises a set of analysis equipment. The method further comprises generating the modified analysis result data corresponding to the analysis results. The method also comprises sending the portions of the modified analysis result data to the recipient nodes.

The analysis instruction data may comprise at least one of the analysis instructions and the recipient node data.

The analysis instruction data may comprise the sample data.

Generating the analysis results may be based on at least a part of the analysis instruction data.

The analysis results may each initially be according to at least one respective initial analysis reference.

At least two analysis results may be according to different initial analysis references.

At least two analysis nodes may generate analysis results according to different initial analysis references. In other words, at least two analysis nodes use different analysis references.

Generating the modified analysis results may be based on the recipient node data. Generating the modified analysis may alternatively or additionally be based on the sample data.

Sending the portions of the modified analysis result data to the recipient nodes may be based on the recipient node data.

Generating the modified analysis result data may comprise generating modified analysis results at least for each analysis result for which the output analysis reference and the initial analysis reference are different.

The method may further comprise for each analysis result, selecting the output analysis reference based on at least one of the analysis instruction data and the recipient node data.

The method may further comprise for each analysis result, selecting the output analysis reference based on the sample data.

Each analysis result may comprise a numeric result value.

Each analysis result may initially be associated with a segment of a respective initial result range.

Each initial result range may be corresponding to an initial analysis reference.

Generating the modified analysis results may comprise for at least some of the analysis results associating the analysis results to corresponding segments of the output result range.

Each output result range may correspond to an output analysis reference.

Generating the modified analysis results may further comprise for the at least some of the analysis results modifying the numeric result value so as to maintain a same relative position in the segment of the output result range as within the segment of the initial result range.

The same relative position may be a position in a same quantile of the distribution of analysis results in the segments of the output result range and the input result range.

Generating the modified analysis results may further comprise for the at least some of the analysis result, transforming the numeric result value from the segment of the initial result range to the segment of the output result range by means of the affine transformation.

Associating the analysis results to the segment of the output result range, respectively, may comprise associating results from a same segment of the initial result range to a same segment of the output result range.

The method may comprise associating results from a same segment of the initial result range to a same segment of the output result range if a number of segments of the output result range equals a number of segments of the initial result range.

Associating the analysis results to the segment of the output result range may comprise associating results from a plurality of segments of the initial result range to one segment of the output result range, preferably if the initial result range comprises more segments than the output result range.

Associating the analysis results to the segment of the output result range, respectively, may also comprise associating results from one or at least one segment of the initial result range to a plurality of segments of the output result range, preferably if the initial result range comprises less segments than the output result range.

Associating the analysis results to the segment of the output result range, respectively, may further comprise associating results from same continuous intervals of the one or at least one segment of the initial result range to same segments of the output result range.

The method may comprise determining the segment of the output result range based on a pair of the initial analysis reference and the output analysis reference and by processing the model data. As indicated, the model data can comprise at least some of the above- features of the model data discussed in the context of the system.

Generating the modified analysis result data may comprise comparing each of the analysis results to the comparison sample group.

Comparing the analysis results to the comparison sample group may comprise estimating the relative position of the respective analysis result in the corresponding comparison sample group.

The relative position in the comparison group may be the position in a quantile of the comparison sample group.

The relative position of analysis result in the comparison group may be a segment of results in the comparison sample group in which the respective analysis result falls.

The method may comprise for each analysis result, selecting the comparison sample group.

Selecting the comparison sample group for each analysis result may be based at least on the sample data.

The sample data may comprise data relating to each of the plurality of samples.

The samples may be samples originating from the human body, such as samples of the bodily fluid or samples of the tissue from the human body.

The analysis results may relate to the bio-medical properties of the samples.

The analysis results may relate to quantitative analyses.

The sample data may comprise the sample identification data of the samples, which sample identification data may identify each sample.

The sample data may comprise user data relating to the users from whom the samples originate.

The user data may comprise the demographic data of the users.

The user data may comprise data relating to the age of the users.

The user data may comprise data relating to the sex of the users.

The user data may comprise genomic data of the users.

The user data may comprise data relating to the ethnicity of the users.

The user data may comprise data relating to the environment of the users.

The user data may comprise data relating to the geographic environment in which the users live.

The user data may comprise data relating to the occupation of the users.

The user data may comprise the portions relating to the existing medical conditions of the respective users.

The user data may comprise the portions relating to the medications of the respective users.

The user data may comprise portions relating to the drug consumption of the respective users.

Selecting the comparison sample group for each analysis result may be based at least on the user data relating to the users from whom the samples originate.

The sample comparison groups may comprise analysis results of samples corresponding to the similar user data.

The similar user data may be similar according to the similarity measure.

At least two of the sample comparison groups may each comprise the analysis results corresponding to the samples from to users of a same sex.

At least two of the sample comparison groups may each comprise the analysis results corresponding to the samples from users of a similar ethnicity.

At least two of the sample comparison groups may each comprise the analysis results corresponding to the samples from users whose user data comprise similar portions relating to the medical conditions.

At least two of the sample comparison groups may each comprise the analysis results corresponding to the samples from users whose user data comprise similar portions relating to the medication.

At least two of the sample comparison groups may each comprise the analysis results corresponding to samples from users whose user data comprise similar portions relating to the drug consumption.

The method may comprise generating, such as determining or estimating, the similarity by means of the similarity measure, at least based on at least one of the demographic data of the users, the data relating to the ethnicity of the users, the data relating to the environment of the users and the data relating existing medical conditions of the users.

The method may comprise generating the similarity by means of the similarity measure, at least based on the data relating to the medications of the users.

The method may comprise generating the similarity by means of the similarity measure, at least based on the data relating to the drug consumption of the users.

The recipient node data may comprise for each sample the indication of the recipient node.

The indications of the recipient node may comprise the indication of the address associated with the corresponding recipient node data-processing system, such as the IP-address, the e-mail address, the ID, the authentication token, the hash of any of these, or the like.

Each recipient node may comprise the corresponding recipient node data-processing system.

The analysis instructions may comprise instructions for analysing each of the samples.

The analysis instructions may comprise instructions for performing at least one bio-medical analysis for each sample.

The analysis results may be bio-medical analysis results.

The method may comprise generating analysis data by means of the sets of analysis equipment.

The sets of analysis equipment may comprise the devices for performing at least one bio-medical analysis, such as the centrifuge or the thermal cycler.

The method may comprise transmitting the analysis data to the corresponding analysis node data processing systems.

The analysis nodes may process the analysis data.

The analysis node data processing systems may process the analysis data.

The analysis nodes may generate the analysis results based on the analysis data.

The method may comprise generating the modified analysis result data by means of the data-processing system.

The modification node may comprise the data-processing system which generates the modified analysis results.

The method may comprise transmitting the analysis results from the analysis nodes to the modification node.

The modification node may transmit the portions of the modified analysis result data to the recipient nodes.

The modification node may comprise the output reference selector component. The output reference selector component may select the output analysis reference.

The modification node may comprise a range association component. The range association component may associate the at least some of the analysis results to the corresponding segments of the output result range.

The modification node may comprise the sample comparison component. The sample comparison component may compare the analysis results to the comparison sample group.

The modification node may comprise a selector node. The selector component may select the comparison sample group for each analysis result.

In a third embodiment, a computer program product is disclosed. Above-disclosed features and advantages may respectively apply.

A computer program product comprises instructions which, when the program is executed by a computer or a computer system, cause the computer or the computer system to carry out the steps of the method that the modification node carries out.

Another computer program product comprises instructions which, when the program is executed by a computer or a computer system, cause the computer or the computer system to carry out the steps for which the modification node is configured.

The following embodiments also form part of the invention.

**System embodiments**

Below, embodiments of a system will be discussed. The system embodiments are abbreviated by the letter "S" followed by a number. Whenever reference is herein made to the "system embodiments", these embodiments are meant.

S1. A system, comprising a modification node (70) and at least two analysis nodes (22, 22').

S2. The system according to the preceding embodiment, wherein the system is configured for receiving analysis instruction data (10, 10', 10").

S3. The system according to the preceding embodiment, wherein the analysis nodes are configured for receiving at least a part of the analysis instruction data (10, 10', 10").

S4. The system according to any of the preceding system embodiments, wherein the analysis nodes are configured for generating analysis results for a plurality of samples.

S5. The system according to any of the preceding system embodiments, wherein each of analysis nodes comprise a set of analysis equipment.

S6. The system according to any of the preceding system embodiments, wherein the modification node (70) is configured for generating modified analysis result data corresponding to the analysis results.

S7. The system according to the preceding embodiment, wherein the system is configured for sending portions of the modified analysis result data to recipient nodes.

S8. The system according to the preceding embodiment, wherein the modification node is configured for sending portions of the modified analysis result data to the recipient nodes.

S9. The system according to any of the preceding system embodiments with the features of S2, wherein the analysis instruction data comprise at least one of analysis instructions (14) and recipient node data (16).

S10. The system according to any of the preceding system embodiments with the features of S2, wherein the analysis instruction data comprise sample data.

S11. The system according to any of the preceding system embodiments with the features of S4 and S2, wherein the analysis nodes are configured for generating the analysis results based on at least a part of the analysis instruction data (10, 10', 10").

S12. The system according to any of the preceding system embodiments with the features of S4, wherein the analysis results generated by the analysis nodes are each initially according to at least one respective initial analysis reference (40, 42, 44, 46).

S13. The system according to the preceding method embodiment, wherein at least two analysis results are according to different initial analysis references.

S14. The system according to any of the two preceding embodiments, wherein each analysis node is configured for using at least one initial analysis reference (40, 42, 44, 46), and wherein at least two analysis nodes are configured for generating analysis results according to different initial analysis references (40, 42, 44, 46).

S15. The system according to any of the preceding embodiments with the features of S6 and at least one of S9 and S10, wherein the modification node is configured for generating the modified analysis results based on at least one of the recipient node data and the sample data.

S16. The system according to any of the preceding embodiments with the features of S9 and at least one of S7 and S8, wherein the system, preferably the modification node, is configured for sending the portions of the modified analysis result data to the recipient nodes based on the recipient node data.

S17. The system according to any of the preceding embodiments with the features of at least one of S5 and S6, wherein the system and/or the modification node are configured for generating modified analysis results at least for each analysis result for which an output analysis reference and the initial analysis reference are different.

S18. The system according to the preceding embodiment and with the features of S9, wherein the system and/or the modification node is further configured for selecting the output analysis reference for each analysis result based on at least one of the analysis instruction data and the recipient node data.

S19. The system according to any of the two preceding system embodiments and with the features of S10, wherein the system and/or the modification node is further configured for selecting the output analysis reference for each analysis result based on the sample data.

S20. The system according to any of the two preceding embodiments, wherein the modification node comprises an output reference selector component, and wherein the output reference selector component is configured for selecting the output analysis reference.

S21. The system according to any of the preceding system embodiments with the features of S4, wherein each analysis result comprises a numeric result value.

S22. The system according to any of the preceding system embodiments with the features of S4, wherein each analysis result is initially associated with a segment of a respective initial result range.

S23. The system according to the preceding embodiment and preferably to S12, wherein each initial result range is corresponding to an initial analysis reference.

S24. The system according to any of the preceding embodiments with the features of S6, wherein generating the modified analysis results comprises for at least some of the analysis results, associating the analysis results to corresponding segments of an output result range.

S25. The system according to the preceding embodiment, wherein the modification node (70) comprises a range association component, and wherein the range association component is configured for associating at least some of the analysis results to the corresponding segments of the output result range.

S26. The system according to any of the two preceding embodiments, wherein each output result range corresponds to an output analysis reference.

S27. The system according to any of the preceding embodiments with the features of S21, S22 and S24, wherein generating the modified analysis results further comprises for the at least some of the analysis results modifying the numeric result value so as to maintain a same relative position in the segment of the output result range as within the segment of the initial result range.

S28. The system according to the preceding embodiment, wherein the same relative position is a position in a same quantile of a distribution of analysis results in the segments of the output result range and the input result range.

S29. The system according to any of the preceding embodiments with the features of S21, S22 and S24, wherein generating the modified analysis results further comprises for the at least some of the analysis results, transforming the numeric result value from the segment of the initial result range to the segment of the output result range by means of an affine transformation.

S30. The system according to any of the preceding embodiments with the features of S21, S22 and S24, wherein the data-processing system and/or the modification node is configured for associating the analysis results to the segment of the output result range, respectively, and for associating results from a same segment of the initial result range to a same segment of the output result range.

S31. The system according to the preceding embodiment, wherein the system and/or the modification node is configured for associating results from a same segment of the initial result range to a same segment of the output result range if a number of segments of the output result range equals a number of segments of the initial result range.

S32. The system according to any of the preceding embodiments with the features of S21, S22 and S24, wherein associating the analysis results to the segments of the output result range, respectively, comprises associating results from a plurality of segments of the initial result range to one segment of the output result range, preferably if the initial result range comprises more segments than the output result range.

S33. The system according to any of the preceding embodiments with the features of S21, S22 and S24, wherein associating the analysis results to the segment of the output result range, respectively, comprises associating results from one or at least one segment of the initial result range to a plurality of segments of the output result range, preferably if the initial result range comprises less segments than the output result range.

S34. The system according to the preceding embodiment, wherein associating the analysis results to the segment of the output result range, respectively, comprises associating results from same continuous intervals of the one or at least one segment of the initial result range to same segments of the output result range.

S35. The system according to any of the preceding embodiments with the features of S23 and S26, wherein the system and/or the modification node is configured for determining the segment of the output result range based on a pair of the initial analysis reference and the output analysis reference and by processing model data.

S36. The system according to any of the preceding embodiments, wherein generating the modified analysis result data comprises comparing each of the analysis results to a comparison sample group.

S37. The system according to the preceding embodiment, wherein the modification node comprises a sample comparison component, and wherein the sample comparison component is configured for comparing each of the analysis results to the comparison sample group.

S38. The system according to any of the two preceding embodiments, wherein comparing the analysis results to the comparison sample group comprises estimating a relative position of the respective analysis result in the corresponding comparison sample group.

S39. The system according to the preceding embodiment, wherein the relative position in the comparison group is a position in a quantile of the comparison sample group.

S40. The system according to any of the two preceding embodiments, wherein the relative position of analysis result in the comparison group is a segment of results in the comparison sample group in which the respective analysis result falls.

S41. The system according to any of the preceding embodiments with the features of S36, wherein the system and/or the modification node is further configured for selecting the comparison sample group for each analysis result.

S42. The system according to the preceding embodiment, wherein the modification node further comprises a selector node, and wherein the selector component is configured for selecting the comparison sample group for each analysis result.

S43. The system according to any of the two preceding embodiments and with the features of S10, wherein selecting the comparison sample group for each analysis result is based at least on the sample data.

S44. The system according to the preceding embodiment, wherein the sample data comprise data relating to each of the plurality of samples.

S45. The system according to any of the preceding embodiments, wherein the samples are samples originating from the human body, such as samples of a bodily fluid or samples of tissue from the human body.

S46. The system according to any of the preceding embodiments with the features of S4, wherein the analysis results relate to bio-medical properties of the samples.

S47. The system according to any of the two preceding embodiments, wherein the analysis results relate to quantitative analyses.

S48. The system according to any of the preceding embodiments with the features of S10, wherein the sample data comprise sample identification data of the samples, which sample identification data may be configured for identifying each sample.

S49. The system according to any of the preceding embodiments with the features of S10 and S45, wherein the sample data comprise user data relating to the users from whom the samples originate.

S50. The system according to any of the preceding embodiments with the features of S49, wherein the user data comprise demographic data of the users.

S51. The system according to any of the preceding embodiments with the features of S49, wherein the user data comprise data relating to an age of the users.

S52. The system according to any of the preceding embodiments with the features of S49, wherein the user data comprise data relating to a sex of the users.

S53. The system according to any of the preceding embodiments with the features of S49, wherein the user data comprise genomic data of the users.

S54. The system according to any of the preceding embodiments with the features of S49, wherein the user data comprise data relating to an ethnicity of the users.

S55. The system according to any of the preceding embodiments with the features of S49, wherein the user data comprise data relating to an environment of the users.

S56. The system according to the preceding embodiment, wherein the user data comprise data relating to a geographic environment in which the users live.

S57. The system according to any of the two preceding embodiments, wherein the user data comprise data relating to an occupation of the users.

S58. The system according to any of the preceding embodiments with the features of S49, wherein the user data comprise portions relating to existing medical conditions of the respective users.

S59. The system according to any of the preceding embodiments with the features of S49, wherein the user data comprise portions relating to medications of the respective users.

S60. The system according to any of the preceding embodiments with the features of S49, wherein the user data comprise portions relating to a drug consumption of the respective users.

S61. The system according to any of the preceding embodiments with the features of S43 and S49, wherein selecting the comparison sample group for each analysis result is based at least on the user data relating to the users from whom the samples originate.

S62. The system according to any of the preceding embodiments with the features of S36 and S49, wherein the sample comparison groups comprise analysis results of samples corresponding to similar user data.

S63. The system according to the preceding embodiment, wherein the similar user data are similar according to a similarity measure.

S64. The system according to the preceding embodiment and with the features of S42, wherein the selector component is configured for applying the similarity measure.

S65. The system according to any of the three preceding embodiments and with the features of S52, wherein at least two of the sample comparison groups each comprise analysis results corresponding to samples from to users of a same sex.

S66. The system according to any of the four preceding embodiments and with the features of S54, wherein at least two of the sample comparison groups each comprise analysis results corresponding to samples from users of a similar ethnicity.

S67. The system according to any of the five preceding embodiments and with the features of S58, wherein at least two of the sample comparison groups each comprise analysis results corresponding to samples from users whose user data comprise similar portions similar medical conditions.

S68. The system according to any of the six preceding system embodiments and with the features of S59, wherein at least two of the sample comparison groups each comprise analysis results corresponding to samples from users whose user data comprise similar portions relating to the medication.

S69. The system according to any of the seven preceding embodiments and with the features of S60, wherein at least two of the sample comparison groups each comprise analysis results corresponding to samples from users whose user data comprise similar portions relating to the drug consumption.

S70. The system according to any of the preceding embodiments and with the features of S63 and S49, wherein the system and/or the modification node is configured for generating the similarity by means of the similarity measure at least based on at least one of the demographic data of the users, the data relating to the ethnicity of the users, the data relating to the environment of the users and the data relating existing medical conditions of the users.

S71. The system according to any of the preceding embodiments and with the features of S63, S49 and S59, wherein the system and/or the modification node is configured for generating the similarity by means of the similarity measure at least based on the data relating to the medications of the users.

S72. The system according to any of the preceding embodiments and with the features of S63, S49 and S60, wherein the system and/or the modification node is configured for generating the similarity by means of the similarity measure at least based on the data relating to the drug consumption of the users.

S73. The system according to any of the preceding system embodiments and with the features of S9 and S4, wherein the recipient node data comprise for each sample an indication of a recipient node.

S74. The system according to the preceding embodiment, wherein the indications of the recipient node comprise an indication of an address associated with a corresponding recipient node data-processing system, such as an IP-address, an e-mail address, an ID, an authentication token, a hash of any of these, or the like.

S75. The system according to any of the preceding embodiments and with the features of S7, wherein each of the recipient nodes comprises the corresponding recipient node data-processing system.

S76. The system according to any of the preceding embodiments and with the features of S7, wherein the system comprises the recipient nodes.

S77. The system according to any of the preceding embodiments and with the features of S9, wherein analysis instructions comprise instructions for analysing each of the samples.

S78. The system according to any of the preceding embodiments and with the features of S9, wherein the analysis instructions comprise instructions for performing at least one bio-medical analysis for each sample.

S79. The system according to any of the preceding embodiments and with the features of S4, wherein the analysis results are bio-medical analysis results.

S80. The system according to any of the preceding embodiments, wherein the sets of analysis equipment are configured for generating analysis data.

S81. The system according to any of the preceding embodiments, wherein the sets of analysis equipment comprise devices configured for performing at least one bio-medical analysis, such as a centrifuge or a thermal cycler.

S82. The system according to any of the preceding embodiments with the features of S4 and S80, wherein each analysis node comprises an analysis node data processing system, and wherein the analysis nodes are configured for transmitting the analysis data to corresponding analysis node data processing systems.

S83. The system according to any of the preceding embodiments with the features of S4, wherein the analysis nodes are configured for processing the analysis data.

S84. The system according to the two preceding embodiments, wherein the analysis node data processing systems are configured for processing the analysis data.

S85. The system according to any of the two preceding embodiments, wherein the analysis nodes are configured for generating the analysis results based on the analysis data.

**Method embodiments**

Below, embodiments of a method will be discussed. The method embodiments are abbreviated by the letter "M" followed by a number. Whenever reference is herein made to the "method embodiments", these embodiments are meant.

M1. A method, comprising

(a) receiving analysis instruction data (10, 10', 10"),
(b) generating analysis results for a plurality of samples by at least two analysis nodes each comprising a set of analysis equipment,
(c) generating modified analysis result data corresponding to the analysis results, and
(d) sending portions of the modified analysis result data to recipient nodes.

M2. The method according to the preceding method embodiment, wherein the analysis instruction data comprise at least one of analysis instructions (14) and recipient node data (16).

M3. The method according to any of the preceding embodiments, wherein the analysis instruction data comprise sample data.

M4. The method according to any of the preceding method embodiments, wherein generating the analysis results is based on at least a part of the analysis instruction data (10, 10', 10").

M5. The method according to any of the preceding method embodiments, wherein the analysis results are each initially according to at least one respective initial analysis reference (40, 42, 44, 46).

M6. The method according to the preceding method embodiment, wherein at least two analysis results are according to different initial analysis references.

M7. The method according to any of the two preceding embodiments, wherein at least two analysis nodes generate analysis results according to different initial analysis references (40, 42, 44, 46).

M8. The method according to any of the preceding method embodiments with the features of at least one of M2 and M3, wherein generating the modified analysis results is based on at least one of the recipient node data and the sample data.

M9. The method according to any of the preceding method embodiments with the features of M2, wherein sending the portions of the modified analysis result data to the recipient nodes is based on the recipient node data.

M10. The method according to any of the preceding method embodiments, wherein generating the modified analysis result data comprises generating modified analysis results at least for each analysis result for which an output analysis reference and the initial analysis reference are different.

M11. The method according to the preceding method embodiment and with the features of M2, wherein the method further comprises for each analysis result, selecting the output analysis reference based on at least one of the analysis instruction data and the recipient node data.

M12. The method according to any of the two preceding method embodiments and with the features of M3, wherein the method further comprises for each analysis result, selecting the output analysis reference based on the sample data.

M13. The method according to any of the preceding method embodiments, wherein each analysis result comprises a numeric result value.

M14. The method according to any of the preceding method embodiments, wherein each analysis result is initially associated with a segment of a respective initial result range.

M15. The method according to the preceding method embodiment, wherein each initial result range is corresponding to an initial analysis reference.

M16. The method according to any of the preceding method embodiments, wherein generating the modified analysis results comprises for at least some of the analysis results associating the analysis results to corresponding segments of an output result range.

M17. The method according to the preceding method embodiment, wherein each output result range corresponds to an output analysis reference.

M18. The method according to any of the preceding method embodiments with the features of M13, M14 and M16, wherein generating the modified analysis results further comprises for the at least some of the analysis results modifying the numeric result value so as to maintain a same relative position in the segment of the output result range as within the segment of the initial result range.

M19. The method according to the preceding method embodiment, wherein the same relative position is a position in a same quantile of a distribution of analysis results in the segments of the output result range and the input result range.

M20. The method according to any of the preceding method embodiments with the features of M13, M14 and M16, wherein generating the modified analysis results further comprises for the at least some of the analysis result, transforming the numeric result value from the segment of the initial result range to the segment of the output result range by means of an affine transformation.

M21. The method according to any of the preceding method embodiments with the features of M13, M14 and M16, wherein associating the analysis results to the segment of the output result range, respectively, comprises associating results from a same segment of the initial result range to a same segment of the output result range.

M22. The method according to the preceding embodiment, wherein the method comprises associating results from a same segment of the initial result range to a same segment of the output result range if a number of segments of the output result range equals a number of segments of the initial result range.

M23. The method according to any of the preceding method embodiments with the features of M13, M14 and M16, wherein associating the analysis results to the segments of the output result range, respectively, comprises associating results from a plurality of segments of the initial result range to one segment of the output result range, preferably if the initial result range comprises more segments than the output result range.

M24. The method according to any of the preceding method embodiments with the features of M13, M14 and M16, wherein associating the analysis results to the segment of the output result range, respectively, comprises associating results from one or at least one segment of the initial result range to a plurality of segments of the output result range, preferably if the initial result range comprises less segments than the output result range.

M25. The method according to the preceding embodiment, wherein associating the analysis results to the segment of the output result range, respectively, comprises associating results from same continuous intervals of the one or at least one segment of the initial result range to same segments of the output result range.

M26. The method according to any of the preceding method embodiments with the features of M15 and M17, wherein the method comprises determining the segment of the output result range based on a pair of the initial analysis reference and the output analysis reference and by processing model data.

M27. The method according to any of the preceding method embodiments, wherein generating the modified analysis result data comprises comparing each of the analysis results to a comparison sample group.

M28. The method according to the preceding method embodiment, wherein comparing the analysis results to the comparison sample group comprises estimating a relative position of the respective analysis result in the corresponding comparison sample group.

M29. The method according to the preceding method embodiment, wherein the relative position in the comparison group is a position in a quantile of the comparison sample group.

M30. The method according to any of the two preceding method embodiments, wherein the relative position of analysis result in the comparison group is a segment of results in the comparison sample group in which the respective analysis result falls.

M31. The method according to any of the preceding method embodiments with the features of M27, wherein the method comprises for each analysis result, selecting the comparison sample group.

M32. The method according to the preceding method embodiment and with the features of M3, wherein selecting the comparison sample group for each analysis result is based at least on the sample data.

M33. The method according to the preceding method embodiment, wherein the sample data comprise data relating to each of the plurality of samples.

M34. The method according to any of the preceding method embodiments, wherein the samples are samples originating from the human body, such as samples of a bodily fluid or samples of tissue from the human body.

M35. The method according to any of the preceding method embodiments, wherein the analysis results relate to bio-medical properties of the samples.

M36. The method according to any of the two preceding method embodiments, wherein the analysis results relate to quantitative analyses.

M37. The method according to any of the preceding method embodiments with the features of M3, wherein the sample data comprise sample identification data of the samples, which sample identification data identify each sample.

M38. The method according to any of the preceding method embodiments with the features of M3 and M34, wherein the sample data comprise user data relating to the users from whom the samples originate.

M39. The method according to any of the preceding method embodiments with the features of M38, wherein the user data comprise demographic data of the users.

M40. The method according to any of the preceding method embodiments with the features of M38, wherein the user data comprise data relating to an age of the users.

M41. The method according to any of the preceding method embodiments with the features of M38, wherein the user data comprise data relating to a sex of the users.

M42. The method according to any of the preceding method embodiments with the features of M38, wherein the user data comprise genomic data of the users.

M43. The method according to any of the preceding method embodiments with the features of M38, wherein the user data comprise data relating to an ethnicity of the users.

M44. The method according to any of the preceding method embodiments with the features of M38, wherein the user data comprise data relating to an environment of the users.

M45. The method according to the preceding method embodiment, wherein the user data comprise data relating to a geographic environment in which the users live.

M46. The method according to any of the two preceding method embodiments, wherein the user data comprise data relating to an occupation of the users.

M47. The method according to any of the preceding method embodiments with the features of M38, wherein the user data comprise portions relating to existing medical conditions of the respective users.

M48. The method according to any of the preceding method embodiments with the features of M38, wherein the user data comprise portions relating to medications of the respective users.

M49. The method according to any of the preceding method embodiments with the features of M38, wherein the user data comprise portions relating to a drug consumption of the respective users.

M50. The method according to any of the preceding method embodiments with the features of M32 and M38, wherein selecting the comparison sample group for each analysis result is based at least on the user data relating to the users from whom the samples originate.

M51. The method according to any of the preceding method embodiments with the features of M27 and M38, wherein the sample comparison groups comprise analysis results of samples corresponding to similar user data.

M52. The method according to the preceding method embodiment, wherein the similar user data are similar according to a similarity measure.

M53. The method according to any of the two preceding method embodiments and with the features of M41, wherein at least two of the sample comparison groups each comprise analysis results corresponding to samples from to users of a same sex.

M54. The method according to any of the three preceding method embodiments and with the features of M43, wherein at least two of the sample comparison groups each comprise analysis results corresponding to samples from users of a similar ethnicity.

M55. The method according to any of the four preceding method embodiments and with the features of M47, wherein at least two of the sample comparison groups each comprise analysis results corresponding to samples from users whose user data comprise similar portions relating to the medical conditions.

M56. The method according to any of the five preceding method embodiments and with the features of M48, wherein at least two of the sample comparison groups each comprise analysis results corresponding to samples from users whose user data comprise similar portions relating to the medication.

M57. The method according to any of the six preceding method embodiments and with the features of M49, wherein at least two of the sample comparison groups each comprise analysis results corresponding to samples from users whose user data comprise similar portions relating to the drug consumption.

M58. The method according to any of the preceding method embodiments and with the features of M52 and M38, wherein the method comprises generating the similarity by means of the similarity measure, at least based on at least one of the demographic data of the users, the data relating to the ethnicity of the users, the data relating to the environment of the users and the data relating existing medical conditions of the users.

M59. The method according to the preceding embodiment and with the features of M48, wherein the method comprises generating the similarity by means of the similarity measure, at least based on the data relating to the medications of the users.

M60. The method according to any of the two preceding embodiments and with the features of M49, wherein the method comprises generating the similarity by means of the similarity measure, at least based on the data relating to the drug consumption of the users.

M61. The method according to any of the preceding method embodiments and with the features of M2, wherein the recipient node data comprise for each sample an indication of a recipient node.

M62. The method according to the preceding embodiment, wherein the indications of the recipient node comprise an indication of an address associated with a corresponding recipient node data-processing system, such as an IP-address, an e-mail address, an ID, an authentication token, a hash of any of these, or the like.

M63. The method according to the preceding embodiment, wherein each recipient node comprises the corresponding recipient node data-processing system.

M64. The method according to any of the preceding method embodiments and with the features of M2, wherein analysis instructions comprise instructions for analysing each of the samples.

M65. The method according to any of the preceding method embodiments and with the features of M2, wherein the analysis instructions comprise instructions for performing at least one bio-medical analysis for each sample.

M66. The method according to any of the preceding method embodiments, wherein the analysis results are bio-medical analysis results.

M67. The method according to any of the preceding method embodiments, wherein the method comprises generating analysis data by means of the sets of analysis equipment.

M68. The method according to any of the preceding method embodiments, wherein the sets of analysis equipment comprise devices for performing at least one bio-medical analysis, such as a centrifuge or a thermal cycler.

M69. The method according to any of the preceding method embodiments with the features of M67, wherein the method comprises transmitting the analysis data to corresponding analysis node data processing systems.

M70. The method according to any of the preceding method embodiments, wherein the analysis nodes process the analysis data.

M71. The method according to the two preceding method embodiments, wherein the analysis node data processing systems process the analysis data.

M72. The method according to any of the two preceding embodiments, wherein the analysis nodes generate the analysis results based on the analysis data.

M73. The method according to any of the preceding method embodiments, wherein the method comprises generating the modified analysis result data by means of a data-processing system.

M74. The method according to the preceding two embodiment, wherein a modification node comprises the data-processing system which generates the modified analysis results.

M75. The method according to the preceding method embodiments, wherein the method comprises transmitting the analysis results from the analysis nodes to a modification node.

M76. The method according to any of the preceding method embodiments and with the features of M74, wherein the modification node transmits the portions of the modified analysis result data to the recipient nodes.

M77. The method according to any of the preceding method embodiments and with the features of M74 and M11, wherein the modification node comprises an output reference selector component, and wherein the output reference selector component selects the output analysis reference.

M78. The method according to any of the preceding method embodiments and with the features of M74 and M16, wherein the modification node comprises a range association component, and wherein the range association component associates the at least some of the analysis results to the corresponding segments of the output result range.

M79. The method according to any of the preceding method embodiments and with the features of M74 and M27, wherein the modification node comprises a sample comparison component, and wherein the sample comparison component compares the analysis results to the comparison sample group.

M80. The method according to any of the preceding method embodiments and with the features of M74 and M31, wherein the modification node comprises a selector node, and wherein the selector component selects for the comparison sample group each analysis result.

**Computer program product embodiments**

Below, embodiments of a computer program product will be discussed. These embodiments are abbreviated by the letter "C" followed by a number. Whenever reference is herein made to the "computer program product embodiments", these embodiments are meant.

P1. A computer program product comprising instructions which, when the program is executed by a computer or a computer system, cause the computer or the computer system to carry out the steps of the method that the modification node carries out.

P.2 A computer program product comprising instructions which, when the program is executed by a computer or a computer system, cause the computer or the computer system to carry out the steps for which the modification node is configured.

Exemplary features of the invention are further detailed in the figures and the below description of the figures.

**Figure description**

Brief description of the figures:

Fig. 1 shows a system and method for sample analysis.
Fig. 2a and Fig. 2b show results and result ranges of measurements.
Fig. 3a and Fig. 3b show different results in different result ranges.

For the sake of clarity, some features may only be shown in some figures, and others may be omitted. However, also the omitted features may be present, and the shown and discussed features do not need to be present in all embodiments.

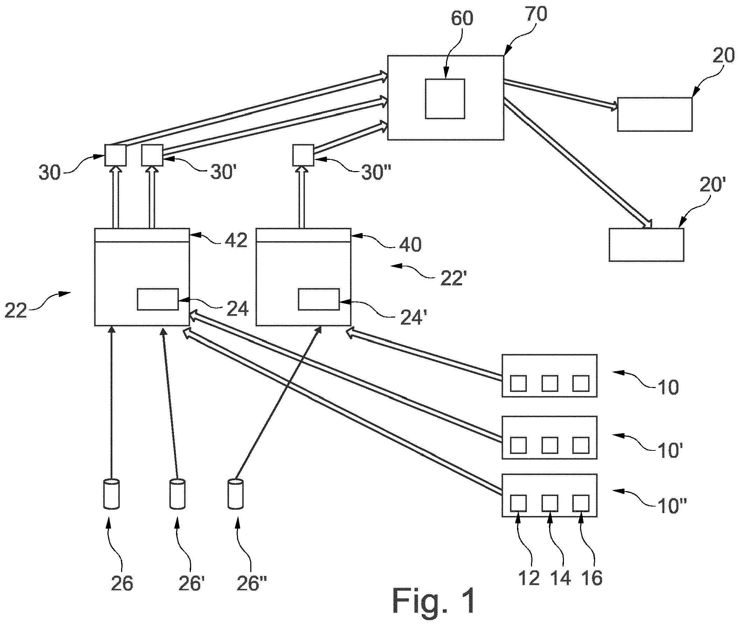

Fig. 1 shows a system and method for sample analysis. Analysis instruction data (10, 10', 10") are sent to analysis nodes (22, 22'). For example, a part of the analysis instruction data 10', 10", may be sent to a first analysis node 22, and another part of the analysis instruction data 10 may be sent to a second analysis node 22'.

The analysis instruction data 10, 10', 10" may comprise sample data 12. The sample data 12 may relate to a sample 26, 26', 26". The sample data 12 may also relate to a plurality of samples 26, 26', 26". For example, the sample data 12 may comprise for each sample 26, 26', 26" a portion relating to said sample. The sample data 12 may comprise identification data which may be configured for identifying the samples 26, 26', 26", such as an identification of a packaging of the sample. For example, an identification number corresponding to a marking of a sample container can be stored. Markings of sample containers are discussed for example in EP 1 803 110.

The analysis instruction data 10, 10', 10" may further comprise at least one or a plurality of analysis instructions 14. The analysis instructions 14 may specify for each sample an analysis to be performed. More precisely, a property to be determined, such as a concentration of low-density lipoprotein (LDL) in a sample 26, 26', 26", may be indicated by the analysis instructions 14. The analysis instructions may further indicate an analysis method or analysis procedure for determining the property. In the example of LDL, different analysis methods are known, which may however result in different quantitative results for a same sample. See for example Miller W. G., Myers G. L., Sakurabayashi I., Bachmann L. M., Caudill S. P., Dziekonski A., Edwards S., Kimberly M. M., Korzun W. J., Leary E. T., Nakajima K., Nakamura M., Nilsson G., Shamburek R. D., Vetrovec G. W., Warnick G. R., Remaley A. T. (2010), "Seven Direct Methods for Measuring HDL and LDL Cholesterol Compared with Ultracentrifugation Reference Measurement Procedures" Clinical Chemistry: Volume 56, Issue 6: 977-986

The analysis instruction data 10, 10', 10" may further comprise recipient node data 16. The recipient node data 16 may indicate a recipient node 20, 20' where a sample 26, 26', 26" was taken. The recipient node data 16 may also indicate a recipient node 20, 20' to which a portion of analysis result data corresponding to the sample is to be sent.

The analysis instruction data 10, 10', 10" may cause the analysis nodes 22, 22' to perform analyses of the corresponding samples 26, 26', 26". The analysis nodes may each comprise a set of analysis equipment 24, 24'. The sets of analysis equipment 24, 24' may comprise analysis equipment for bio-medical analyses of samples. For example, the sets of analysis equipment 24, 24' may comprise devices such as centrifuges for centrifugation of blood, thermal cyclers configured for performing PCR-analyses, microscopes, and like devices well known in the art.

The analysis nodes 22, 22' may generate analysis results 30, 30', 30". The analysis results 30, 30', 30" may be in some parts specific to the analysis nodes 22, 22', for example to the respective set of analysis equipment 24, 24'. For example a calibration of the sets of analysis equipment or the analysis nodes may influence a result. Also, comparison values, such as a standard population for determining whether a measure or a property of a sample is for example in a "normal" range, may be specific to the analysis nodes 22, 22' or the sets of analysis equipment 24, 24'.

The analysis nodes 22, 22' may each comprise a data processing system, particularly for generating the analysis results based on analyses performed by means of the sets of analysis equipment 24, 24'. The data processing systems of the analysis nodes may for example be configured for performing a functionality of a laboratory information system. The analysis nodes 22, 22' can for example be medical or bio-medical laboratories.

The data processing systems of the analysis nodes 22, 22' may for example comprise means of data processing, such as, processor units, hardware accelerators and/or microcontrollers. The data processing systems of the analysis nodes 22, 22' may comprise memory components, such as, main memory (e.g. RAM), cache memory (e.g. SRAM) and/or secondary memory (e.g. HDD, SDD). The data processing systems of the analysis nodes 22, 22' may comprise busses configured to facilitate data exchange between components of the data processing systems, such as, the communication between the memory components and the processing components. The data processing systems of the analysis nodes 22, 22' can comprise network interface cards that can be configured to connect the data processing systems to a network, such as, to the Internet. The data processing systems of the analysis nodes 22, 22' can comprise user interfaces, such as:

an output user interface, such as screens or monitors configured to display visual data and/or speakers configured to communicate audio data, and

an input user interface, such as a camera configured to capture visual data, a microphone configured to capture audio data, a keyboard configured to allow the insertion of text and/or other keyboard commands and/or a mouse, touchscreen and/or trackpad - configured to facilitate the navigation through different graphical user interfaces.

The data processing systems of the analysis nodes 22, 22' may also comprise an interface to the respective set of analysis equipment, for example by means of a local area network.

The analysis nodes 22, 22' may generate analysis results 30, 30', 30" for the samples 26, 26', 26". The analysis results 30, 30', 30" may be respectively be according to an analysis reference 40, 42, 44, 46. The analysis reference may for example indicate an analysis procedure or method, as discussed above. The analysis reference may also indicate comparison values for properties of samples, such as the above discussed "normal" range.

The analysis nodes 22, 22' may send the generated analysis results 30, 30', 30" to a modification node 70. The modification node 70 may comprise a data-processing system. The modification node 70 may store model data 60.

The modification node 70 may be configured for generating modified analysis result data. The modified analysis result data may be generated based on the recipient node data 16 corresponding to the sample to which a respective analysis result 30, 30', 30" relates. More particularly, for an analysis result 30, 30', 30", modified analysis result data may be generated which are according to another analysis reference 40, 42, 44, 46 than the initial analysis result.

This can for example be optionally advantageous if the analysis result data are used afterwards in a context where analysis results according to a first analysis reference 40 are used. In such cases, it would normally be necessary to have the analysis results generated according to said first analysis reference 40. However, if an analysis result of a sample is required in two different contexts, for example by different end users, such as MDs, where typically, different analysis references 40, 42, 44, 46 are used, this leads to a problem. The problem can be resolved by generating analysis results for a same sample (or two samples from a same origin) twice according to two different analysis references 40, 42, 44, 46. However, this can require the resources and/or time for two analyses instead of one. Hence, generating modified analysis result data can be optionally advantageous, as it may save further analyses and the respective resources/time.

The modified analysis result data may additionally or alternatively be generated based on the sample data 12 corresponding to the sample to which a respective analysis result 30, 30', 30" relates.

For example, the modified analysis result data may be generated based on a sex of a user from whom the sample originates. This may optionally advantageous, as some bio-medical properties of samples may be differently distributed in a population among the different sexes. Thus, an analysis result indicating a relative position of a property of a sample in a comparison group and/or a deviation from a normal value or normal range of values may thus be more precise.

In another example, the modified analysis result data may be generated based on an ethnicity of a user from whom the sample originates. This may be optionally advantageous, as it has been shown that some bio-medical values of "healthy" or "normal" users vary depending on their ethnicity. See for example Grann, V.R., Bowman, N., Joseph, C., Wei, Y., Horwitz, M.S., Jacobson, J.S., Santella, R.P. and Hershman, D.L. (2008), "Neutropenia in 6 ethnic groups from the Caribbean and the U.S." Cancer, 113: 854-860 as well as LIM, E.-M., CEMBROWSKI, G., CEMBROWSKI, M. and CLARKE, G. (2010), "Race-specific WBC and neutrophil count reference intervals." International Journal of Laboratory Hematology, 32: 590-597.

Thus, an analysis result indicating a relative position of a property of a sample in a comparison group and/or a deviation from a normal value or normal range of values may be more precise.

The data-processing system of the modification node 70 can comprise means of data processing, such as, processor units, hardware accelerators and/or microcontrollers. The data-processing system of the modification node 70 can comprise memory components, such as, main memory (e.g. RAM), cache memory (e.g. SRAM) and/or secondary memory (e.g. HDD, SDD). The data-processing system of the modification node 70 can comprise busses configured to facilitate data exchange between components of the data-processing system, such as, the communication between the memory components and the processing components. The data-processing system of the modification node 70 can comprise network interface cards that can be configured to connect the data processing device to a network, such as, to the Internet. The data-processing system of the modification node 70 can comprise user interfaces, such as an output user interface, such as screens or monitors configured to display visual data, speakers configured to communicate audio data (e.g. playing audio data to the user). The data-processing system of the modification node 70 can also comprise an input user interface, such as a camera configured to capture visual data, a microphone configured to capture audio data (e.g. recording audio from the user), a keyboard configured to allow the insertion of text and/or other keyboard commands (e.g. allowing the user to enter text data and/or other keyboard commands by having the user type on the keyboard) and/or a trackpad, a mouse, a touchscreen and/or a joystick.

To put it simply, the data-processing system of the modification node 70 can be a processing unit configured to carry out instructions of a program. The data-processing system of the modification node 70 can be a system-on-chip comprising processing units, memory components and busses. The data-processing system of the modification node 70 can be a personal computer, a laptop, a pocket computer, a smartphone, a tablet computer. The data-processing system of the modification node 70 can be a server. The data-processing system of the modification node 70 can be a processing unit or a system-on-chip that can be interfaced with a personal computer, a laptop, a pocket computer, a smartphone, a tablet computer and/or user interfaces (such as the upper-mentioned user interfaces). The data-processing system of the modification node 70 can be a server system, or a portion of a server system, such as a cloud computing system.

The data-processing system of the modification node 70 can comprise at least a temporary connection to the analysis nodes 22, 22', for example by means of the above-discussed network card. The data-processing system of the modification node 70 can further comprise at least a temporary connection to the recipient nodes 20, 20'.

The modification node 70 can be a portion of the analysis nodes 22, 22'. Alternatively or additionally, the modification node 70 can be a portion of the recipient nodes 20, 20'. For example, modification node 70 can be implemented in software, and the recipient nodes and/or the analysis nodes 22, 22' execute this software. In another example, the modification node 70 can also comprise at least one computing component connected to at least one of the analysis nodes 22, 22'. The modification node 70 can also comprise at least one computing component connected to at least one of the recipient nodes 20, 20'.

The person ordinarily skilled in the art will easily understand that in some cases, a portion of the modified analysis result data may comprise a portion of the analysis results 30, 30', 30" in their original form or in an unmodified form, particularly if the original form is the form in which this part of the modified analysis result data is needed afterwards.

The recipient nodes 20, 20' can receive portions of the modified analysis result data. The recipient nodes 20, 20' may each comprise a data-processing system as discussed above in the context of the data-processing system of the modification node 70 and the data-processing systems of the analysis-nodes 22, 22'.

The recipient nodes 20, 20' can output at least a port of the portion of the modified analysis result data that they receive. The recipient nodes 20, 20' can for example comprise data-processing systems configured for patient management at a health care provider.

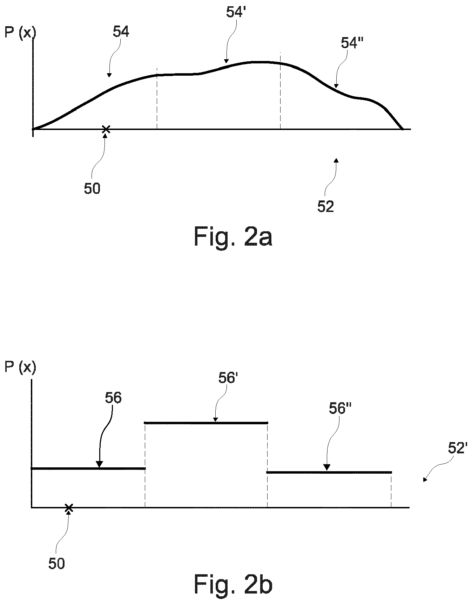

Fig. 2a and 2b show a numeric result value 50 of an analysis result 30, 30', 30" in different result ranges 52, 52'. The result ranges may each be associated with one or more analysis references 40, 42, 44, 46.

In Fig. 2a, the numeric value 50 of the analysis result 30, 30', 30" is shown in a first result range 52. The result range comprises three segments: a left (low) segment 54, 56, a middle (normal) segment 54', 56' and a right (high) segment 54", 56". On the y-axis, an estimation of a probability density function of the numeric value 50, for example estimated by a number of occurrences, is shown. As discussed above, said density function (as well as its estimation) may vary depending on origins of a sample 26, 26', 26".

The result range 52 in Fig. 2a may be associated with a first calibration of an analysis equipment 24, 24' of an analysis node 22, 22'. Thus, the limits of the of the range segments 54, 54', 54" may be specific to the calibration. As an example in Fig. 2a, 1/3 of the results in the low range segment are below the numeric result value 50 and 2/3 of the results in the low range segment are above the numeric result value 50.

Fig. 2b shows the numeric result value 50 corresponding to modified analysis result data. These modified result data can for example be according to a second analysis reference 42, whereas the initial analysis result 30, 30', 30" can for example have been according to a first analysis reference 40.

As can be seen, the modification has been performed so as to maintain a relative position of the analysis result in a comparison group - again, 1/3 of the results in the low range segment 56 are below the numeric result value 50, and 2/3 of the results in the low range segment are above the numeric result value 50.

This can be optionally advantageous particularly in cases where the estimated densities can be applied to the modified analysis result, for example if they represent a density of analysis results in a general representative population, or for female or male users only. In such cases, the modified analysis result data comprise a value which has a same significance as the value in the initial analysis result 30, 30', 30". Thus, ergonomics of at least a portion of the modified analysis result data can be improved in comparison to other conversion method.

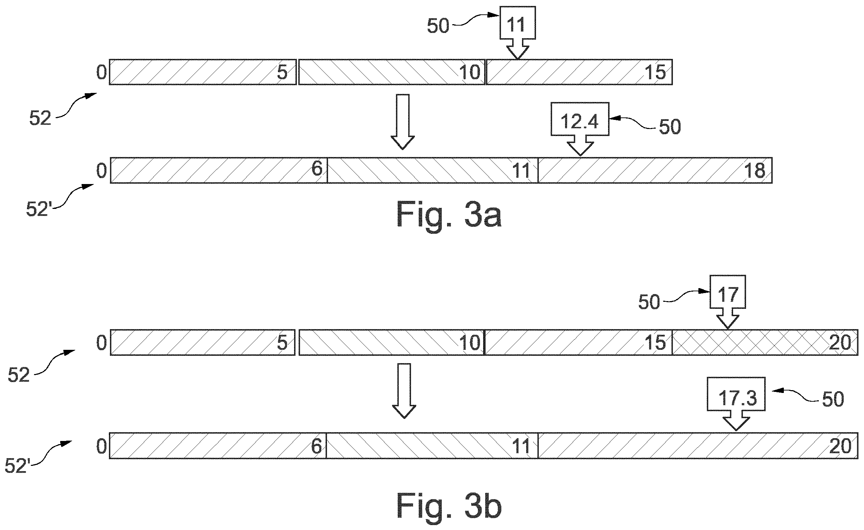

Fig. 3a and Fig. 3b show another example of generating modified analysis result data.

In Fig. 3a, an affine transformation for generating the modified analysis result data is shown. As in Fig 2a and 2b, two result ranges 52, 52' can be seen. As in the constellation in Fig 2a and 2b, the result ranges may have a same number of segments 54, 54', 54", 56, 56', 56".

In some cases, it may be difficult to estimate the density of results shown in Fig. 2a and 2b, for example due to a lack of data or due to privacy concerns.

Fig. 3a shows an alternative, comprising generating the modified analysis result data by means of an affine transformation, associating each numeric value 50 in each segment 54, 54', 54" of one result range 52 to a numeric value 50 in a segment 54, 54', 54" of the other result range 52'.

In some cases, as discussed above, for an ergonomic interpretation of the modified analysis result data, it may be optionally advantageous to associate numeric values 50 from one segment 54 of the first result range 52 to numeric values 50 from only one segment 54 of the second result range 52'. For example, numeric values 50 which were initially classified as being "high" (by lying inside a "high" segment) of the first result range 52, may only be associated to values of the "high" segment of the second result range 52'.

This may further be optionally advantageous in cases where the result ranges 52, 52' and corresponding analysis references 40, 42, 44, 46 differ because of a different scale of a generated measurement and/or because of linear influences. In such cases, an affine transformation may require only few data sets and pre-calculation compared to a statistical analysis.

$$y = (x - a)\frac{d - c}{b - a} + c$$

Eq. 1

An affine transformation can for example be performed by means of eq. 1, wherein y is a numeric value 50 corresponding to a segment of the second result range 52', x is a numeric value 50 corresponding to a segment of the first result range 52, a and b are a lower and an upper limit of the segment of the first result range and c and d a lower and an upper limit of the segment of the second result range.

Fig. 3b shows a situation where a number of segments of the result ranges 52, 52' is different.

As a mere example, a first result range 52 in Fig. 3b comprises four segments, which can for example be associated to "low", "medium", "high" and "very high" values. In the same example, a second result range 52' in Fig. 3b can comprise three segments.

The person ordinarily skilled in the art will easily understand that generally, the segments 54, 54', 54", 56, 56', 56" of the result ranges do not need to each comprise an interval of a same size, but that they can be of different size. This can also apply to corresponding intervals.

In the case shown in Fig. 3b, a same affine transformation as for the case in Fig. 3a can be performed. In such a case, two segments of the first result range 52 can be considered as one result range. In the example of Fig. 3b, this can be a segment comprising an interval [10, 15] and a segment comprising an interval [15, 20]. This unified segment can then be used for generating the modified analysis result data.

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

**Numbered references**

| | |
|---|---|
| 10, 10', 10" | Analysis instruction data |
| 12 | Sample data |
| 14 | Analysis instruction |
| 16 | Recipient node data |
| 20, 20' | Recipient node |
| 22, 22' | Analysis node |
| 24, 24' | Set of analysis equipment |
| 26, 26', 26" | Sample |
| 30, 30', 30" | Analysis result |
| 40, 42, 44, 46 | Analysis reference |
| 50 | Numeric result value |
| 52, 52' | Result range |
| 54, 54', 54", 56, 56', 56" | Segment |
| 60 | Model data |

70                               Modification node

**Claims**

1. A system, comprising a modification node (70) and at least two analysis nodes (22, 22'), wherein the system is configured for receiving analysis instruction data (10, 10', 10"),
   wherein the analysis nodes are configured for generating analysis results for a plurality of samples,
   wherein the modification node (70) is configured for generating modified analysis result data corresponding to the analysis results, and for sending portions of the modified analysis result data to recipient nodes.

2. The system according to the preceding claim, wherein the modification node comprises a sample comparison component,

   wherein generating the modified analysis result data comprises comparing each of the analysis results to a comparison sample group,
   wherein comparing the analysis results to the comparison sample group comprises estimating a relative position of the respective analysis result in the corresponding comparison sample group, and
   wherein the sample comparison component is configured for comparing each of the analysis results to the comparison sample group, and wherein the modification node further comprises a selector node, and wherein the selector component is configured for selecting the comparison sample group for each analysis result based at least on sample data.

3. The system according to any of the preceding claims, wherein the samples are samples originating from the human body, wherein the sample data comprise user data relating to users from whom the samples originate, wherein the user data comprise data relating to at least one of a sex of the users, an ethnicity of the users and existing medical conditions of the respective users, and wherein the user data comprise portions relating to at least one of medications of the respective users and a drug consumption of the respective users.

4. The system according to any of claims 2-3, wherein selecting the comparison sample group for each analysis result is based at least on the user data relating to the users from whom the samples originate.

5. The system according to any of the three preceding claims, wherein the sample comparison groups comprise analysis results of samples corresponding to similar user data, wherein the similar user data are similar according to a similarity measure, and wherein the selector component is configured for applying the similarity measure, and wherein the modification node is configured for generating the similarity by means of the similarity measure at least based on at least one of the demographic data of the users, the data relating to the ethnicity of the users, the data relating to the environment of the users, the data relating existing medical conditions of the users, the data relating to the medications of the users and the data relating to the drug consumption of the users.

6. The system according to any of the preceding claims,

   wherein the modification node comprises an output reference selector component,
   wherein the modification node is configured for generating modified analysis results at least for each analysis result for which an output analysis reference and an initial analysis reference are different, and
   wherein the output reference selector component is further configured for selecting the output analysis reference for each analysis result based on at least one of the analysis instruction data, recipient node data and sample data.

7. The system according to any of the preceding claims,

   wherein the modification node (70) comprises a range association component,
   wherein each analysis result comprises a numeric result value and wherein each analysis result is initially associated with a segment of a respective initial result range,
   wherein generating the modified analysis results comprises for at least some of the analysis results, associating the analysis results to corresponding segments of an output result range,
   wherein the range association component is configured for said associating the at least some of the analysis results to the corresponding segments of the output result range, and wherein generating the modified analysis results further comprises for the at least some of the analysis results modifying the numeric result value so as to maintain a same relative position in the segment of the output result range as within the segment of the initial result range.

8. The system according to the preceding claim, wherein

(a) the range association component is configured for associating results from a same segment of the initial result range to a same segment of the output result range if a number of segments of the output result range equals a number of segments of the initial result range,
(b) wherein associating the analysis results to the segments of the output result range, respectively, comprises associating results from a plurality of segments of the initial result range to one segment of the output result range, if the initial result range comprises more segments than the output result range, and/or
(c) wherein associating the analysis results to the segment of the output result range, respectively, comprises associating results from one or at least one segment of the initial result range to a plurality of segments of the output result range, preferably if the initial result range comprises less segments than the output result range.

9. A method, comprising

(a) receiving analysis instruction data (10, 10', 10"),
(b) generating analysis results for a plurality of samples by at least two analysis nodes each comprising a set of analysis equipment,
(c) generating modified analysis result data corresponding to the analysis results, and
(d) sending portions of the modified analysis result data to recipient nodes,

wherein generating the modified analysis result data comprises comparing each of the analysis results to a comparison sample group, wherein comparing the analysis results to the comparison sample group comprises estimating a relative position of the respective analysis result in the corresponding comparison sample group, and wherein the relative position in the comparison group is a position in a quantile of the comparison sample group, wherein the method comprises for each analysis result, selecting the comparison sample group based at least on the sample data., wherein the samples are samples originating from the human body, and sample data comprise user data relating to the users from whom the samples originate, wherein the user data comprise data relating to at least one of a sex of the users, an ethnicity of the users, existing medical conditions of the respective users, medications of the respective users and a drug consumption of the respective users.

10. The method according to the preceding claim, wherein selecting the comparison sample group for each analysis result is based at least on the user data relating to the users from whom the samples originate.

11. The method according to any of the two preceding claims, wherein the sample comparison groups comprise analysis results of samples corresponding to similar user data, wherein the similar user data are similar according to a similarity measure, wherein the method comprises generating the similarity by means of the similarity measure, at least based on at least one of the demographic data of the users, the data relating to the ethnicity of the users, the data relating to the environment of the users, the data relating existing medical conditions of the users, the data relating to the medications of the users and the data relating to the drug consumption of the users.

12. The method according to any of the preceding claims 9-11, wherein generating the modified analysis result data comprises generating modified analysis results at least for each analysis result for which an output analysis reference and an initial analysis reference are different, wherein the method further comprises for each analysis result, selecting the output analysis reference based on at least one of the analysis instruction data, the recipient node data and the sample data.

13. The method according to any of the preceding claims 9-12, wherein each analysis result comprises a numeric result value and wherein each analysis result is initially associated with a segment of a respective initial result range, and wherein generating the modified analysis results comprises for at least some of the analysis results associating the analysis results to corresponding segments of an output result range, wherein generating the modified analysis results further comprises for the at least some of the analysis results modifying the numeric result value so as to maintain a same relative position in the segment of the output result range as within the segment of the initial result range.

14. The method according to the preceding claim, wherein

(a) the method comprises associating results from a same segment of the initial result range to a same segment of the output result range if a number of segments of the output result range equals a number of segments of the initial result range,

(b) wherein associating the analysis results to the segments of the output result range, respectively, comprises associating results from a plurality of segments of the initial result range to one segment of the output result range, preferably if the initial result range comprises more segments than the output result range, and/or

(c) wherein associating the analysis results to the segment of the output result range, respectively, comprises associating results from one or at least one segment of the initial result range to a plurality of segments of the output result range, preferably if the initial result range comprises less segments than the output result range.

15. A computer program product comprising instructions which, when the program is executed by a computer or a computer system, cause the computer or the computer system to carry out the method according to any of the claims 8-14.

Fig. 1

Fig. 2a

Fig. 2b

Fig. 3a

Fig. 3b

Europäisches Patentamt

European Patent Office

Office européen des brevets

## EUROPEAN SEARCH REPORT

Application Number

EP 20 18 2720

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/050116 A1 (BLICK KENNETH E [US]) 25 February 2010 (2010-02-25) * The whole document, in particular: Paragraphs [0004], [0012], [0013], [0020] - [0026], [0039], [0052], [0067]; Figure 1. * | 1-15 | INV. G16H10/40 G16H10/60 |
| X | US 2020/090801 A1 (SCOTT CARY [US] ET AL) 19 March 2020 (2020-03-19) * The whole document, in particular: Paragraphs [0004], [0006], [0011], [0047] - [0052]; Figures 1, 9A. * | 1-15 | |
| X | US 2020/082946 A1 (KATAYEV ALEXANDER L [US] ET AL) 12 March 2020 (2020-03-12) * The whole document, in particular: Paragraphs [0003], [0004], [0008], [0010], [0034] - [0037], [0045], [0046], [0054], [0059], [0097], [0100]; Figures 1 - 3. * | 1-15 | |
| A | US 2018/308581 A1 (SOTTAS PIERRE-EDOUARD [CH] ET AL) 25 October 2018 (2018-10-25) * The whole document, in particular: Paragraphs [0002] - [0007]; Claims 1 - 3. * | 1-15 | TECHNICAL FIELDS SEARCHED (IPC) G16H |

The present search report has been drawn up for all claims

| Place of search | Date of completion of the search | Examiner |
|---|---|---|
| Munich | 16 November 2020 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 2720

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2020

| Patent document cited in search report | | Publication date | Patent family member(s) | | Publication date |
|---|---|---|---|---|---|
| US 2010050116 | A1 | 25-02-2010 | CN | 102124468 A | 13-07-2011 |
| | | | JP | 2012500403 A | 05-01-2012 |
| | | | US | 2010050116 A1 | 25-02-2010 |
| | | | WO | 2010022047 A2 | 25-02-2010 |
| US 2020090801 | A1 | 19-03-2020 | US | 2020090801 A1 | 19-03-2020 |
| | | | WO | 2020061066 A1 | 26-03-2020 |
| US 2020082946 | A1 | 12-03-2020 | CA | 2900393 A1 | 28-08-2014 |
| | | | EP | 2959414 A1 | 30-12-2015 |
| | | | HK | 1218014 A1 | 27-01-2017 |
| | | | US | 2014236491 A1 | 21-08-2014 |
| | | | US | 2020082946 A1 | 12-03-2020 |
| | | | WO | 2014130562 A1 | 28-08-2014 |
| US 2018308581 | A1 | 25-10-2018 | CN | 108604464 A | 28-09-2018 |
| | | | EP | 3365818 A1 | 29-08-2018 |
| | | | JP | 2018536846 A | 13-12-2018 |
| | | | US | 2018308581 A1 | 25-10-2018 |
| | | | WO | 2017068146 A1 | 27-04-2017 |

EPO FORM P0459

For more details about this annex : see Official Journal of the European Patent Office, No. 12/82

## REFERENCES CITED IN THE DESCRIPTION

### Patent documents cited in the description

- WO 9805427 A
- EP 180311 A
- EP 1803110 A

### Non-patent literature cited in the description

- **MILLER W. G. ; MYERS G. L. ; SAKURABAYASHI I. ; BACHMANN L. M. ; CAUDILL S. P. ; DZIEKONSKI A. ; EDWARDS S. ; KIMBERLY M. M. ; KORZUN W. J. ; LEARY E. T.** Seven Direct Methods for Measuring HDL and LDL Cholesterol Compared with Ultracentrifugation Reference Measurement Procedures. *Clinical Chemistry,* 2010, vol. 56 (6), 977-986
- **GRANN, V.R. ; BOWMAN, N. ; JOSEPH, C. ; WEI, Y. ; HORWITZ, M.S. ; JACOBSON, J.S. ; SANTELLA, R.P. ; HERSHMAN, D.L.** Neutropenia in 6 ethnic groups from the Caribbean and the U.S. *Cancer,* 2008, vol. 113, 854-860
- **LIM, E.-M. ; CEMBROWSKI, G. ; CEMBROWSKI, M. ; CLARKE, G.** Race-specific WBC and neutrophil count reference intervals. *International Journal of Laboratory Hematology,* 2010, vol. 32, 590-597